# EUROPEAN PATENT APPLICATION

(11) **EP 4 252 771 A1**
(43) Date of publication of application: **04.10.2023**
(21) Application number: 21898137.1
(22) Date of filing: 29.11.2021
(51) Int. Cl.: A61K 39/395, A61P 17/00

(54) **INHIBITOR OF FIBROSIS PROGRESSION**

(30) Priority: 30.11.2020 WO PCT/JP2020/044406
(71) Applicant: Maruho Co., Ltd., Osaka 531-0071 (JP); The University of Tokyo, Bunkyo-ku, Tokyo 113-8654 (JP)
(72) Inventor: YOSHIZAKI, Ayumi, Tokyo 113-8654 (JP); KUZUMI, Ai, Tokyo 113-8654 (JP); SATO, Shinichi, Tokyo 113-8654 (JP); FUJITA, Tomoyuki, Osaka-shi, Osaka 531-0071 (JP); WATANABE, Hideki, Osaka-shi, Osaka 531-0071 (JP)
(74) Representative: Vossius & Partner Patentanwälte Rechtsanwälte mbB
(86) International application number: PCT/JP2021/043543
(87) International publication number: WO 2022/114165

(57) **Abstract**

In one embodiment, the present disclosure provides pharmaceutical compositions for suppressing the progression of fibrosis in systemic sclerosis, which contain an antibody against IL-31 receptor A as an active ingredient. In another embodiment, the present disclosure provides pharmaceutical compositions for suppressing Th2 polarization to suppress the progression of fibrosis in systemic sclerosis, which comprise an antibody against IL-31 receptor A as an active ingredient. In a certain embodiment, the above-mentioned antibody is an antibody having a neutralizing activity against IL-31 receptor A.

## Description

### [Technical Field]

The present disclosure relates to pharmaceutical compositions comprising an antibody against IL-31 receptor A as an active ingredient. In one embodiment, the present disclosure relates to agents for suppressing the progression of fibrosis in systemic sclerosis, which comprise an antibody against IL-31 receptor A as an active ingredient. In one embodiment, the present disclosure relates to Th2 polarization-suppressing agents for suppressing the progression of fibrosis in systemic sclerosis, which comprise an antibody against IL-31 receptor A as an active ingredient.

### [Background Art]

Systemic sclerosis (SSc) is a connective tissue disease characterized by excessive deposition of extracellular matrix such as collagen in the skin and internal organs (Non-Patent Literatures (NPLs) 1 and 2). The fibrosis resulting from the excessive deposition leads to tissue dysfunction and organ failure that can be debilitating and life threatening for patients. Although the pathogenesis of SSc still remains unknown, three abnormalities-autoimmunity, angiopathy and fibrosis-are greatly involved. Activation and polarization (bias, shift) of T cells have been extensively studied both in patients and in animal models of SSc (NPL 3). For example, CD4+ T cells have been shown to infiltrate the lesional skin during the early stage of SSc (NPL 4). These infiltrating T cells show high expression of activation markers (NPL 5). T cells in the peripheral blood have also been found to be activated (NPL 6). In addition, activated CD4+ T cells in SSc are predominantly skewed to T helper (Th) 2 (NPLs 3 and 7). Indeed, major Th2 cytokines (cytokines produced from Th2 cells) such as interleukin (IL)-4, IL-6, and IL-13 are overexpressed in the skin and serum of SSc patients (NPLs 8-11). Th2 cytokines are reported to be also associated with skin and lung fibrosis in bleomycin-induced SSc model (BLM-SSc) mice, a known SSc-like animal model (NPLs 12-13). Mechanistically, these Th2 cytokines directly induce collagen production in fibroblasts (NPLs 14-16). Moreover, IL-4 and IL-6 drive the differentiation of naive CD4+ T cells into Th2 cells, thus perpetuating the Th2 and pro-fibrotic responses (NPLs 17-18). Taken together, these studies suggest that Th2 dominance is a key immunological feature of SSc that promotes fibrosis.

Factors whose association with Th2-dominant diseases has been suggested include IL-31, an IL-6 family Th2 cytokine, in addition to IL-4, IL-6, and IL-13. Specifically, it has been shown that IL-31 is highly expressed in Th2-dominant diseases such as allergic asthma, atopic dermatitis, and cutaneous T-cell lymphoma (NPLs 19-24). In the context of SSc, it has been reported that IL-31 expression is increased in fibrotic lungs of BLM-SSc mice (NPL 25).

Excessive production of extracellular matrices such as collagen by fibroblasts and their excessive deposition are known as cardinal pathological features of SSc, and this leads to progressive fibrosis of the skin and internal organs (NPL 26). There are few reports of studies on the role of IL-31 in the excessive production and excessive deposition of collagen and/or such in SSc, but recent reports have shown that IL-31 stimulation promoted collagen production by dermal fibroblasts from healthy individuals (NPLs 27 and 28). In these reports, however, IL-31 stimulation did not increase collagen production in dermal fibroblasts from SSc patients. Thus, the role of IL-31 in fibrosis is still not clear. Neither is there any report confirming that the administration of an anti-IL-31 receptor A antibody can suppress the progression of fibrosis.

### [Citation List]

### [Non-Patent Literature]

[NPL 1] Gabrielli A, Avvedimento EV, Krieg T. Scleroderma. N Engl J Med 2009; 360: 1989-2003.
[NPL 2] Yoshizaki A. Pathogenic roles of B lymphocytes in systemic sclerosis. Immunol Lett 2018; 195: 76-82.
[NPL 3] O'Reilly S, Hugle T, van Laar JM. T cells in systemic sclerosis: a reappraisal. Rheumatology (Oxford) 2012; 51: 1540-9.
[NPL 4] Roumm AD, Whiteside TL, Medsger TA, et al. Lymphocytes in the skin of patients with progressive systemic sclerosis. Quantification, subtyping, and clinical correlations. Arthritis Rheum 1984; 27: 645-53.
[NPL 5] Kalogerou A, Gelou E, Mountantonakis S, et al. Early T cell activation in the skin from patients with systemic sclerosis. Ann Rheum Dis 2005; 64: 1233-5.
[NPL 6] Fiocco U, Rosada M, Cozzi L, et al. Early phenotypic activation of circulating helper memory T cells in scleroderma: correlation with disease activity. Ann Rheum Dis 1993; 52: 272-7.
[NPL 7] Barron L, Wynn TA. Fibrosis is regulated by Th2 and Th17 responses and by dynamic interactions between fibroblasts and macrophages. Am J Gastrointest Liver Physiol 2011; 300: G723-8.
[NPL 8] Sato S, Hasegawa M, Takehara K. Serum levels of interleukin-6 and interleukin-10 correlate with total skin thickness score in patients with systemic sclerosis. J Dermatol Sci 2001; 27: 140-6.
[NPL 9] Hasegawa M, Fujimoto M, Kikuchi K, et al. Elevated serum levels of interleukin 4 (IL-4), IL-10, and IL-13 in patients with systemic sclerosis. J Rheumatol 1997; 24: 328-32.
[NPL 10] Salmon-Ehr V, Serpier H, Nawrocki B, et al. Expression of interleukin-4 in scleroderma skin specimens and scleroderma fibroblast cultures. Potential role in fibrosis. Arch Dermatol 1996; 132: 802-6.
[NPL 11] Koch AE, Kronfeld-Harrington LB, Szekanecz Z, et al. In situ expression of cytokines and cellular adhesion molecules in the skin of patients with systemic sclerosis. Pathobiology 1993; 61: 239-46.
[NPL 12] Yoshizaki A, Yanaba K, Iwata Y, et al. Cell Adhesion Molecules Regulate Fibrotic Process via Th1/Th2/Th17 Cell Balance in a Bleomycin-Induced Scleroderma Model. J Immunol 2010; 185: 2502-15.
[NPL 13] Yamamoto T, Takagawa S, Katayama I, et al. Animal model of sclerotic skin. I: Local injections of bleomycin induce sclerotic skin mimicking scleroderma. J Invest Dermatol 1999; 112: 456-62.
[NPL 14] Postlethwaite AE, Holness MA, Katai H, et al. Human fibroblasts synthesize elevated levels of extracellular matrix proteins in response to interleukin 4. J Clin Invest 1992; 90: 1479-85.
[NPL 15] O'Reilly S, Ciechomska M, Cant R, et al. Interleukin-6 (IL-6) trans signaling drives a STAT3-dependent pathway that leads to hyperactive transforming growth factor-β (TGF-β) signaling promoting SMAD3 activation and fibrosis via Gremlin protein. J Biol Chem 2014;289:9952-60.
[NPL 16] Lee CG, Homer RJ, Zhu Z, et al. Interleukin-13 induces tissue fibrosis by selectively stimulating and activating transforming growth factor β1. J Exp Med 2001; 194: 809-21.
[NPL 17] Kopf M, Le GG, Bachmann M, et al. Disruption of the murine IL-4 gene blocks Th2 cytokine responses. Nature 1993; 362: 245-8.
[NPL 18] Rincon M, Anguita J, Nakamura T, et al. Interleukin (IL)-6 directs the differentiation of IL-4-producing CD4+ T cells. J Exp Med 1997; 185: 461-9.
[NPL 19] Lei Z, Liu G, Huang Q, et al. SCF and IL-31 rather than IL-17 and BAFF are potential indicators in patients with allergic asthma. Allergy 2008; 63: 327-32.
[NPL 20] Lai T, Wu D, Li W, et al. Interleukin-31 expression and relation to disease severity in human asthma. Sci Rep 2016; 6: 22835.
[NPL 21] Neis MM, Peters B, Dreuw A, et al. Enhanced expression levels of IL-31 correlate with IL-4 and IL-13 in atopic and allergic contact dermatitis. J Allergy Clin Immunol 2006; 118: 930-7.
[NPL 22] Raap U, Wichmann K, Bruder M, et al. Correlation of IL-31 serum levels with severity of atopic dermatitis. J Allergy Clin Immunol 2008; 122: 421-3.
[NPL 23] Ohmatsu H, Sugaya M, Suga H, et al. Serum IL-31 levels are increased in patients with cutaneous T-cell lymphoma. Acta Derm Venereol 2012; 92: 282-3.
[NPL 24] Nattkemper LA, Martinez-Escala ME, Gelman AB, et al. Cutaneous T-cell Lymphoma and Pruritus: The Expression of IL-31 and its Receptors in the Skin. Acta Derm Venereol 2016; 96: 894-8.
[NPL 25] Shi K, Jiang J, Ma T, et al. Pathogenesis pathways of idiopathic pulmonary fibrosis in bleomycin-induced lung injury model in mice. Respir Physiol Neurobiol 2014;190:113-7.
[NPL 26] Yoshizaki A, Yanaba K, Ogawa A, et al. The specific free radical scavenger edaravone suppresses fibrosis in the bleomycin-induced and tight skin mouse models of systemic sclerosis. Arthritis Rheum 2011; 63: 3086-97.
[NPL 27] Yaseen B, Lopez H, Taki Z, et al. Interleukin-31 promotes pathogenic mechanisms underlying skin and lung fibrosis in scleroderma. Rheumatology (Oxford). [Epub ahead of print: 4 May 2020].
[NPL 28] Taki Z, Gostjeva E, Thilly W, et al. Pathogenic Activation of Mesenchymal Stem Cells is induced by the Disease Microenvironment in Systemic Sclerosis. Arthritis Rheum [Epub ahead of print: 31 Mar 2020].

### [Summary of Invention]

### [Technical Problem]

The invention in the present disclosure has been made in view of the above-mentioned circumstances. In one embodiment, an objective of the invention is to provide a novel means to suppress the progression of fibrosis. In another embodiment, an objective of the invention in the present disclosure is to provide a novel means to suppress Th2 polarization, another key characteristic of the progression of fibrosis in systemic sclerosis.

### [Solution to Problem]

As a result of dedicated research, the present inventors discovered that the administration of an anti-IL-31 receptor A blocking antibody to systemic sclerosis model animals suppresses the progression of fibrosis. The present inventors further discovered that the administration of an anti-IL-31 receptor A antibody suppresses the Th2 polarization of T cells.

The present disclosure is based on these findings, and specifically includes embodiments exemplarily described below.
[A1] An agent for suppressing progression of fibrosis in systemic sclerosis, comprising an antibody against IL-31 receptor A as an active ingredient.
[A2] The agent for suppressing progression of fibrosis according to [A1], which suppresses Th2 polarization.
[A3] The agent for suppressing progression of fibrosis according to [A1] or [A2], wherein the antibody is an antibody having a neutralizing activity against IL-31 receptor A.
[A4] The agent for suppressing progression of fibrosis according to any one of [A1] to [A3], wherein the antibody is:
   (1) an antibody comprising a heavy chain variable region comprising CDR1 having the amino acid sequence of SEQ ID NO: 1, CDR2 having the amino acid sequence of SEQ ID NO: 2, and CDR3 having the amino acid sequence of SEQ ID NO: 3, and a light chain variable region comprising CDR1 having the amino acid sequence of SEQ ID NO: 4, CDR2 having the amino acid sequence of SEQ ID NO: 5, and CDR3 having the amino acid sequence of SEQ ID NO: 6;
   (2) an antibody comprising a heavy chain variable region having the amino acid sequence of SEQ ID NO: 7 and a light chain variable region having the amino acid sequence of SEQ ID NO: 8; or
   (3) an antibody comprising a heavy chain having the amino acid sequence of SEQ ID NO: 9 and a light chain having the amino acid sequence of SEQ ID NO: 10.
[B1] An agent for suppressing Th2 polarization, which comprises an antibody against IL-31 receptor A as an active ingredient, and suppresses progression of fibrosis in systemic sclerosis.
[B2] The agent for suppressing Th2 polarization according to [B1], wherein the antibody is an antibody having a neutralizing activity against IL-31 receptor A.
[D1] A pharmaceutical composition for suppressing progression of fibrosis in systemic sclerosis, comprising an antibody against IL-31 receptor A as an active ingredient.
[D2] A pharmaceutical composition for suppressing Th2 polarization, which comprises an antibody against IL-31 receptor A as an active ingredient and is for suppressing progression of fibrosis in systemic sclerosis.
[D3] The pharmaceutical composition according to [D1] or [D2], wherein the antibody is an antibody having a neutralizing activity against IL-31 receptor A.
[E1] A method for suppressing progression of fibrosis in systemic sclerosis, comprising administering an antibody against IL-31 receptor A.
[E2] A method for suppressing Th2 polarization to suppress progression of fibrosis in systemic sclerosis, comprising administering an antibody against IL-31 receptor A.
[E3] The method according to [E1] or [E2], wherein the antibody is an antibody having a neutralizing activity against IL-31 receptor A.
[F1] An antibody against IL-31 receptor A for use in suppressing progression of fibrosis in systemic sclerosis.
[F2] An antibody against IL-31 receptor A for use in suppressing Th2 polarization to suppress progression of fibrosis in systemic sclerosis.
[F3] The antibody according to [F1] or [F2], wherein the antibody is an antibody having a neutralizing activity against IL-31 receptor A.
[G1] Use of an antibody against IL-31 receptor A in manufacture of an agent for suppressing progression of fibrosis in systemic sclerosis.
[G2] Use of an antibody against IL-31 receptor A in manufacture of a Th2 polarization-suppressing agent for suppressing progression of fibrosis in systemic sclerosis.
[G3] The use according to [G1] or [G2], wherein the antibody is an antibody having a neutralizing activity against IL-31 receptor A.

### [Brief Description of Drawings]

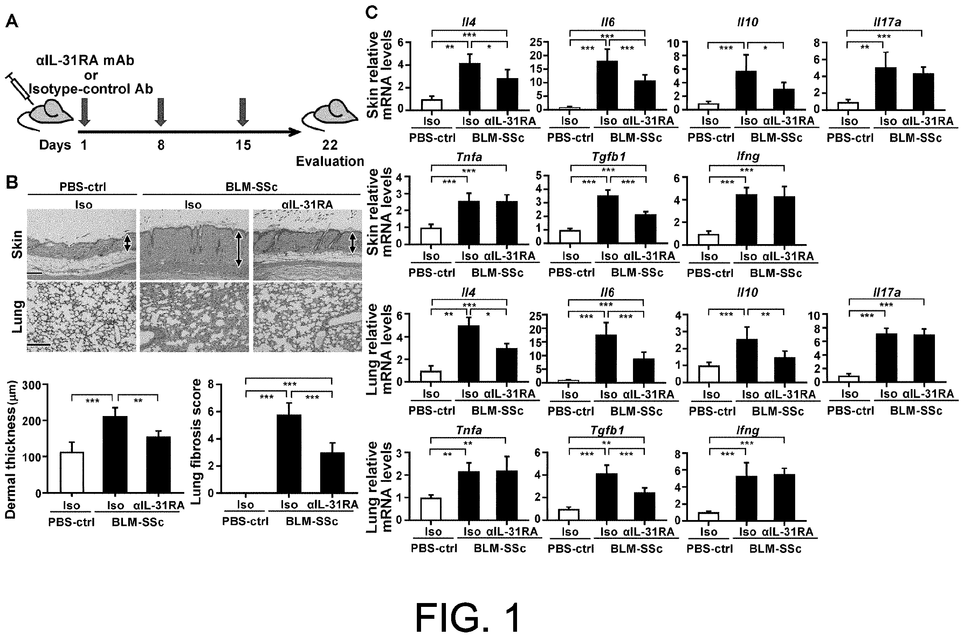

Fig. 1 shows that anti-IL-31RA antibody attenuated the progression of fibrosis induced in BLM-SSc mice. (A) BLM-SSc and PBS-treated control (PBS-ctrl) mice (n = 5 in each group) were administrated with anti-mouse IL-31RA antibody (αIL-31RA) or isotype control IgG (Iso) on days 1, 8, and 15, and then were sacrificed on day 22 for evaluation. (B) The dermal thickness and lung fibrosis score were examined using histological sections obtained from each mouse. Representative histological sections stained with hematoxylin and eosin of the skin and lungs were shown (horizontal scale bars = 100 µm). Vertical bars with arrows represent dermal thickness. (C) The mRNA expression of IL-4, IL-6, IL-10, IL-17A, TNF-α, TGF-β1, and IFN-γ in the skin and lungs of these mice was evaluated by real-time PCR. The bar graphs show the mean + standard deviation. ^{∗}p<0.05, ^{∗∗}p<0.01, and ^{∗∗∗}p<0.001.

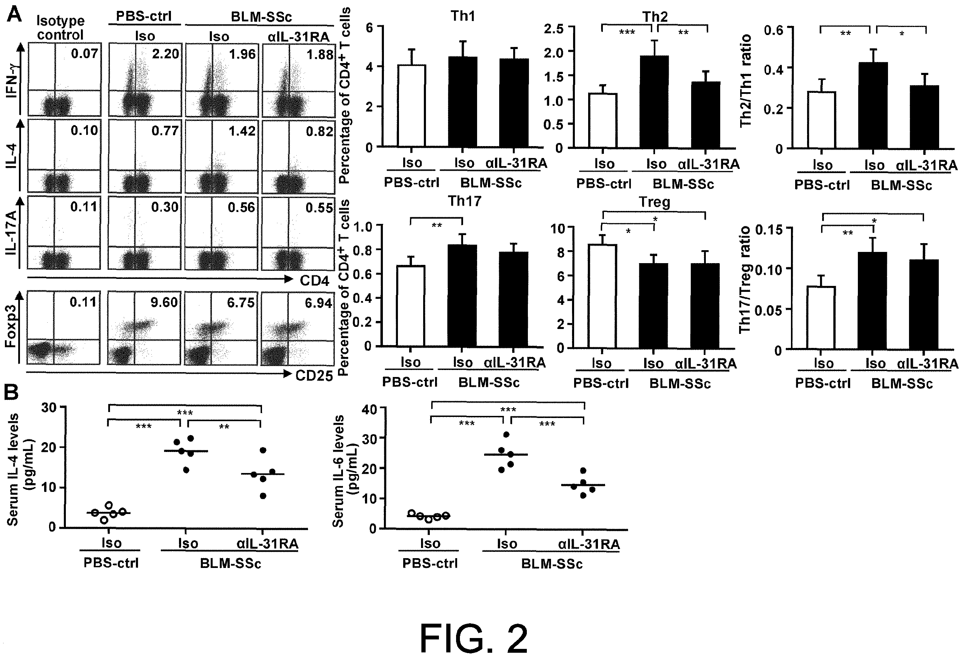

Fig. 2 shows that anti-IL-31RA antibody attenuated the Th2 polarization in BLM-SSc mice. (A) The percentages of splenic Th1, Th2, Th17, and Treg cells in BLM-SSc and PBS-treated control (PBS-ctrl) mice administrated with anti-mouse IL-31RA antibody (αIL-31RA) or isotype control IgG (Iso) were analyzed by flow cytometry. After splenocytes were gated on CD3⁺ populations, IFN-γ⁺CD4⁺ cells were identified as Th1 cells, IL-4⁺CD4⁺ cells as Th2 cells, and IL-17A⁺CD4⁺ cells as Th17 cells. Among the cells gated on CD3⁺CD4⁺ populations, CD25⁺Foxp3⁺ cells were considered as Treg cells. The bar graphs show the mean + standard deviation. (B) The serum levels of IL-4 and IL-6 were assessed by specific ELISA assays. Symbols represent individual mice. The horizontal lines represent the mean values. *p<0.05, **p<0.01, and ***p<0.001.

### [Description of Embodiments]

In one embodiment, the present disclosure provides a pharmaceutical composition for suppressing the progression of fibrosis in systemic sclerosis, and a pharmaceutical composition for suppressing Th2 polarization to suppress the progression of fibrosis in systemic sclerosis (herein also referred to as an agent for suppressing the progression of fibrosis in systemic sclerosis and a Th2 polarization-suppressing agent for suppressing the progression of fibrosis in systemic sclerosis, respectively), which comprise an antibody against IL-31 receptor A as an active ingredient.

In one embodiment, the pharmaceutical composition of the present disclosure can suppress the progression of fibrosis in systemic sclerosis. For example, it can suppress the progression of sclerosis and/or the progression of fibrosis in the skin and internal organs (e.g., lung). In one embodiment, the pharmaceutical composition of the present disclosure can suppress Th2 polarization in systemic sclerosis. For example, it can suppress the production of Th2 cytokines in systemic sclerosis, and/or the progression of a Th2 dominant state in systemic sclerosis.

A pathological abnormality in which fibrosis occurs is called "fibrotic disease". Depending on the tissue where fibrosis occurs, it is called skin fibrosis, lung fibrosis, liver fibrosis, or such. In "systemic sclerosis (SSc)", this fibrosis occurs in the skin and internal organs.

It is known that CD4⁺ T cells play an important role in the development of SSc and infiltrate skin lesions during the early stage of SSc. CD4⁺ T cells are known to have subtypes such as Th1 cells, Th2 cells, Th17 cells, regulatory T cells (Tregs). Each subtype is known to have its characteristic cytokine secretion pattern. Especially, Th1 cells and Th2 cells maintain the homeostasis of the living body by regulating the functions of each other and keeping them balanced. It is said that when this balance (Th1/Th2 balance) is shifted toward either subtype, diseases specific to each subtype will occur. A shift of the Th1/Th2 balance toward Th1 dominance is called "Th1 polarization", and a shift toward Th2 dominance is called "Th2 polarization". It is also said that not only an imbalance between Th1 cells and Th2 cells but also imbalances among the CD4⁺ T cell subtypes including Th17 and Treg cells are involved in disease development. Meanwhile, "Th2 polarization" can also be expressed as "bias toward the Th2 type" or "a shift toward the Th2 type immune response". Similar expressions are also possible for the polarization toward the other CD4+ T cell subtypes.

Herein, "fibrosis" refers to a phenomenon in which the skin or an internal organ becomes stiff as a result of deposition of extracellular matrices such as collagen in the skin or internal organ. It is known that once fibrosis has occurred, fibrosed parts will never return to original soft tissues.

Herein, the phrases "suppressing the progression of fibrosis" and "suppression of fibrosis progression" refer to suppressing or inhibiting the progression of fibrosis compared to a control (e.g., a subject or a group of subjects to whom or which a fibrosis progression-suppressing agent of the present disclosure is not administered).

Various methods for assessing the degree of fibrosis are known. Fibrosis in the skin can be assessed, for example, based on skin thickness in a skin tissue sample observed under a microscope. The severity of fibrosis in the lung can be assessed, for example, by using a lung fibrosis score known as Ashcroft score.

Herein, the phrases "suppressing Th2 polarization" and "suppression of Th2 polarization" refer to suppressing or inhibiting a shift of the Th1/Th2 balance toward Th2 dominance compared to a control. The degree of suppression of Th2 polarization is not limited. For example, a shift of the Th1/Th2 balance toward Th2 dominance may be suppressed, inhibited, or reduced by 10% or more, 20% or more, 30% or more, 40% or more, 50% or more, 60% or more, 70% or more, 80% or more, 90% or more, or 100%, as compared to a control. In one embodiment, a control refers to the degree of Th2 polarization in a subject or a group of subjects (*e.g.,* a systemic sclerosis patient or a group of systemic sclerosis patients with Th2 dominant diseases) to whom or which a Th2 polarization-suppressing agent for suppressing the progression of fibrosis in systemic sclerosis of the present disclosure (a pharmaceutical composition of the present disclosure) is not administered. Alternatively, a shift of the Th1/Th2 balance toward Th2 dominance may be suppressed, inhibited, or reduced to a level equal or similar to that of healthy individuals, for example, to 2.0 times, 1.9 times, 1.8 times, 1.7 times, 1.6 times, 1.5 times, 1.4 times, 1.3 times, 1.2 times, 1.1 times, or 1.0 times the level of healthy individuals.

Methods for assessing the degree of Th1 polarization or Th2 polarization of Th cells (helper T cells) are known. For example, it can be assessed by measuring the expression levels of cytokines specific to each of Th1 and Th2 cells in a biological sample obtained from a subject, and calculating their ratio. Similarly, Th17/Treg polarization can be assessed by calculating the ratio between the expression levels of cytokines specific to each of Th17 and Treg cells.

Herein, the phrases "suppressing the progression of fibrosis in systemic sclerosis" and "suppression of fibrosis progression in systemic sclerosis" refer to suppressing or inhibiting the progression of systemic sclerosis as compared to a control. In one embodiment, a control refers to the severity of systemic sclerosis in a subject or a group of subjects (*e.g.,* a systemic sclerosis patient or a group of systemic sclerosis patients) to whom or which an agent for suppressing the progression of fibrosis in systemic sclerosis of the present disclosure (a pharmaceutical composition of the present disclosure) is not administered. Methods for assessing the degree of progression of fibrosis in systemic sclerosis are known. For example, it can be assessed based on the degree of fibrosis in the skin and/or an internal organ (*e.g.,* lung), the degree of Th2 polarization, or a combination thereof.

"IL-31" is a new member of IL-6 cytokine family that was originally reported as an inducer of dermatitis in mice. IL-31 is mainly produced by Th2 cells and is expressed in a variety of cells, including fibroblasts, keratinocytes, and macrophages. Binding of IL-31 to the IL-31 receptor complex on cell surface activates JAK/STAT, PI3K/AKT, and other intracellular signaling pathways, leading to a wide range of immune responses.

The IL-31 receptor complex is a heterodimer consisting of "IL-31 receptor A (IL-31RA)" and "oncostatin M receptor". IL-31RA is unique to the IL-31 receptor, whereas oncostatin M receptor is shared by a receptor complex for oncostatin M. Within these two receptor subunits, IL-31 binds predominantly to IL-31RA.

Herein, the terms "anti-IL-31 receptor A antibody" and "antibody against IL-31 receptor A" are used synonymously and refer to an antibody capable of specifically binding to IL-31 receptor A (IL-31RA). In the context of the invention of the present disclosure, an antibody against IL-31 receptor A is preferably an antibody that has a neutralizing activity against IL-31 receptor A. Herein, a "neutralizing activity against IL-31 receptor A" is an activity of inhibiting the binding of IL-31 receptor A with its ligand, IL-31, and preferably is an activity of suppressing a biological activity based on IL-31 receptor A. Therefore, an "antibody having a neutralizing activity against IL-31 receptor A" can inhibit the binding of IL-31RA with IL-31 and thereby suppress, inhibit, or block intracellular signaling caused *via* IL-31RA.

Herein, an "antibody" refers to a molecule that specifically binds to a certain antigen determinant (epitope). Antibodies include various antibody structures, including monoclonal antibodies (mAb), polyclonal antibodies (pAb), and antibody fragments, but are not limited thereto.

In the context of the invention of the present disclosure, an antibody against IL-31RA is preferably an antibody against mammalian IL-31RA, and more preferably an antibody against human IL-31RA.

Neutralizing antibodies against human IL-31RA include, for example, Antibody A. Antibody A has been shown to improve the symptoms of atopic dermatitis in clinical trials. Antibody A comprises a heavy chain variable region comprising CDR1 having the amino acid sequence of SEQ ID NO: 1, CDR2 having the amino acid sequence of SEQ ID NO: 2, and CDR3 having the amino acid sequence of SEQ ID NO: 3, and a light chain variable region comprising CDR1 having the amino acid sequence of SEQ ID NO: 4, CDR2 having the amino acid sequence of SEQ ID NO: 5, and CDR3 having the amino acid sequence of SEQ ID NO: 6. Antibody A comprises a heavy chain variable region having the amino acid sequence of SEQ ID NO: 7 and a light chain variable region having the amino acid sequence of SEQ ID NO: 8. Antibody A comprises a heavy chain having the amino acid sequence of SEQ ID NO: 9 and a light chain having the amino acid sequence of SEQ ID NO: 10.

Neutralizing antibodies against mouse IL-31RA include, for example, an anti-mouse IL-31 receptor A function blocking monoclonal antibody comprising a heavy chain variable region having the amino acid sequence of SEQ ID NO: 11 and a light chain variable region having the amino acid sequence of SEQ ID NO: 12. The nucleotide sequences of these heavy chain and light chain variable regions are registered as accession numbers LC554895 and LC554896, respectively, on the DDBJ/EMBL/GenBank International Nucleotide Sequence Database.

All prior art references cited herein are incorporated by reference in their entirety.

### [Examples]

Herein below, the present disclosure will be described in detail with reference to the Examples, but it is not to be construed as being limited thereto.

### Mice and experimental protocol

Wild-type C57BL/6 mice were purchased from The Jackson Laboratory (Bar Harbor, ME, USA). Bleomycin (BLM; Nippon Kayaku, Tokyo, Japan) was dissolved in phosphate-buffered saline (PBS) at a concentration of 1 mg/ml. To generate the BLM-induced SSc model (BLM-SSc) mice, 200 µg of BLM was injected subcutaneously into the shaved backs of female mice daily, as previously described (Yoshizaki A, Iwata Y, Komura K, et al. CD19 regulates skin and lung fibrosis via Toll-like receptor signaling in a model of bleomycin-induced scleroderma. Am J Pathol 2008; 172: 1650-63.). The mice treated with PBS instead of BLM (PBS-ctrl mice) were used as controls for BLM-SSc mice. To evaluate the effects of blocking IL-31 signaling, the functionally blocking anti-mouse IL-31 receptor A monoclonal antibody (the heavy chain variable region: SEQ ID NO: 11; the light chain variable region: SEQ ID NO: 12; registered as DDBJ/EMBL/GenBank accession numbers LC554895, LC554896, respectively), or mouse IgG1 kappa isotype control (eBioscience, San Diego, CA, USA) was injected at a dose of 200 µg intraperitoneally every 7th days (day 1, 8, and 15). All mice used in the study were six weeks old. Five mice per group were examined.

### ELISA

Serum samples were frozen at -80°C until they were used for assays. Serum levels of IL-4 and IL-6 in mice were determined using ELISA kits (R&D Systems). All experiments were performed in accordance with the manufacturers' instructions.

### Histological analysis

Skin and lung tissues were formalin-fixed, embedded in paraffin, and stained with hematoxylin and eosin for histological evaluation. Dermal thickness, defined as the distance between the epidermal-dermal junction and the dermal-adipose junction, was measured. The severity of lung fibrosis was semi-quantitatively assessed, as described by Ashcroft *et al.* (Ashcroft T, Simpson JM, Timbrell V. Simple method of estimating severity of pulmonary fibrosis on a numerical scale. J Clin Pathol 1988; 41: 467-70.). Briefly, the grading criteria were as follows: grade 0 = normal lung; grade 1 = minimal fibrous thickening of alveolar or bronchiolar walls; grade 3 = moderate thickening of walls without obvious damage to lung architecture; grade 5 = increased fibrosis with definite damage to lung structure and formation of fibrous bands or small fibrous masses; grade 7 = severe distortion of structure and large fibrous areas; and grade 8 = total fibrous obliteration of fields. Grades 2, 4, and 6 were used as intermediate pictures between the aforementioned criteria.

### RNA isolation and real-time polymerase chain reaction (PCR)

Total RNA was isolated from tissues using RNeasy spin columns (Qiagen, Crawley, UK), according to the manufacturers' instructions. Total RNA from each sample was reverse-transcribed into cDNA. Gene expression was quantified by SYBR green real-time PCR on an ABI Prism 7000 sequence detector (Applied Biosystems, Foster City, CA, USA). To normalize the amounts of loaded cDNA, GAPDH was used as an internal control. Relative fold differences were calculated using the comparative Ct method as previously described (Yoshizaki A, Iwata Y, Komura K, et al. CD19 regulates skin and lung fibrosis via Toll-like receptor signaling in a model of bleomycin-induced scleroderma. Am J Pathol 2008; 172: 1650-63.). The sequences of primers were as follows:
*Il4-*forward 5'-CAACGAAGAACACCACAGAG-3' (SEQ ID NO: 13),
*Il4*-reverse 5'- GGACTTGGACTCATTCATGG-3' (SEQ ID NO: 14);
*Il6*-forward 5'- GATGGATGCTACCAAACTGGAT-3' (SEQ ID NO: 15),
*Il6*-reverse 5'- CCAGGTAGCTATGGTACTCCAGA-3' (SEQ ID NO: 16);
*Ifng*-forward 5'-TCAAGTGGCATAGATGTGGAAGAA-3' (SEQ ID NO: 17),
*Ifng*-reverse 5'-TGGCTCTGCAGGATTTTCATG-3' (SEQ ID NO: 18);
*Il17a*-forward 5'-CAGCAGCGATCATCCCTCAAAG-3' (SEQ ID NO: 19),
*Il17a*-reverse 5'-CAGGACCAGGATCTCTTGCTG-3' (SEQ ID NO: 20),
*Il10*-forward 5'-TTTGAATTCCCTGGGTGAGAA-3' (SEQ ID NO: 21);
*Il10-*reverse 5'-ACAGGGGAGAAATCGATGACA-3' (SEQ ID NO: 22);
*Tgfb1*-forward 5'-GCAACATGTGGAACTCTACCAGAA-3' (SEQ ID NO: 23),
*Tgfb1*-reverse 5'-GACGTCAAAAGACAGCCACTCA-3' (SEQ ID NO: 24);
*Tnfa*-forward 5'-ACCCTCACACTCAGATCATCTTC-3' (SEQ ID NO: 25),
*Tnfa*-reverse 5'-TGGTGGTTTGCTACGACGT-3' (SEQ ID NO: 26); and
*Gapdh*-forward 5'-CGTGTTCCTACCCCCAATGT-3' (SEQ ID NO: 27),
*Gapdh*-reverse 5'-TGTCATCATACTTGGCA GGTTTCT-3' (SEQ ID NO: 28).

### Flow Cytometry

CD4⁺ T cell differentiation was analyzed by flow cytometry, as previously described (Wen X, He L, Chi Y, et al. Dynamics of Th17 cells and their role in Schistosoma japonicum infection in C57BL/6 mice. PLoS Negl Trop Dis 2011; 5: e1399.). Briefly, for detection of Th1, Th2 or Th17 cells, splenocytes were suspended at 2 × 10⁶ /ml in RPMI 1640 medium and were stimulated with 25 ng/ml PMA (Adipogen, San Diego, CA, USA) and 1 mg/ml ionomycin (Sigma, St Louis, MO, USA) in the presence of 2 µM Monensin (Invitrogen, Carlsbad, CA, USA) for 6 hours at 37°C in 5% CO₂. Samples were then surface stained with anti-CD3-PE mAb and anti-CD4-FITC mAb (eBioscience). After fixation and permeabilization with Cytofix/Cytoperm buffer (BD PharMingen, San Diego, CA, USA), samples were intracellularly stained with APC-conjugated mAbs against IFN-y, IL-4, or IL-17A (eBioscience) for detection of Th1, Th2 or Th17 cells, respectively. APC-conjugated isotype-matched mAbs (eBioscience) were used as controls. For detection of Treg cells, the Mouse Regulatory T Cell Staining Kit (eBioscience) was used as the manufacturer's protocol. Briefly, splenocytes suspended at 2 × 10⁶ /ml were surface stained with anti-CD3-PE/Cy7 mAb, anti-CD4-FITC mAb, and anti-CD25-APC mAb (eBioscience), followed by fixation and permeabilization of cell membranes with Cytofix/Cytoperm and intracellular staining with anti-Foxp3-PE mAb or rat IgG2a-PE as a control. Samples were analyzed with a FACS Verse flow cytometer (BD Biosciences, San Diego, CA, USA).

### Statistical analysis

Data are expressed as means ± standard deviation. Statistical analysis was performed by Mann-Whitney *U* test for two-group comparison. P values less than 0.05 were considered significant. All analyses were performed using GraphPad Prism 7.03 (GraphPad Software, La Jolla, CA, USA).

### Functionally blocking anti-IL-31RA mAb attenuated fibrosis and Th2 polarization in BLM-SSc mice.

To determine the role of IL-31 in the development of SSc, fibrosis and Th2 polarization of BLM-SSc mice treated with functionally blocking anti-IL-31RA mAb were assessed. Anti-IL-31RA mAb was administered weekly along with daily subcutaneous injections of BLM for three weeks (Fig. 1A). Anti-IL-31RA mAb significantly reduced the dermal thickness of BLM-SSc mice compared to isotype control IgG (p<0.01; Fig. 1B). Anti-IL-31RA mAb also had a marked reduction effect of lung fibrosis score in BLM-SSc mice (p<0.001; Fig. 1B). As for cytokine mRNA expression in skin and lung tissues, anti-IL-31RA mAb attenuated the expression of IL-4, IL-6, IL-10, and TGF-β1 that was up-regulated in BLM-SSc mice (Fig. 1C). Furthermore, anti-IL-31RA mAb significantly reduced the percentage of Th2 cells (p<0.01) and Th2/Th1 ratio (p<0.05), but not Th17/Treg ratio in splenic T cells of BLM-SSc mice (Fig. 2A). Anti-IL-31RA mAb also ameliorated the overproduction of serum IL-4 and IL-6 in BLM-SSc mice (p<0.01 and p<0.001, respectively; Fig. 2B). Overall, anti-IL-31RA mAb significantly attenuated BLM-induced fibrosis and Th2 polarization.

### [Industrial Applicability]

The invention of the present disclosure elucidated the effect of administration of an anti-IL-31RA antibody in systemic sclerosis fibrosis model animals. In particular, the pharmaceutical compositions of the present disclosure comprising an anti-IL-31RA antibody suppress the progression of fibrosis in, for example, the skin and lung, and are therefore useful as agents for suppressing the progression of fibrosis in systemic sclerosis. Furthermore, the pharmaceutical compositions of the present disclosure comprising an anti-IL-31RA antibody suppress Th2 polarization, and are therefore useful as Th2 polarization-suppressing agents for suppressing the progression of fibrosis in systemic sclerosis.

## Claims

1. An agent for suppressing progression of fibrosis in systemic sclerosis, comprising an antibody against IL-31 receptor A as an active ingredient.

2. The agent for suppressing progression of fibrosis according to claim 1, which suppresses Th2 polarization.

3. The agent for suppressing progression of fibrosis according to claim 1 or 2, wherein the antibody is an antibody having a neutralizing activity against IL-31 receptor A.

4. The agent for suppressing progression of fibrosis according to any one of claims 1 to 3, wherein the antibody is:
(1) an antibody comprising a heavy chain variable region comprising CDR1 having the amino acid sequence of SEQ ID NO: 1, CDR2 having the amino acid sequence of SEQ ID NO: 2, and CDR3 having the amino acid sequence of SEQ ID NO: 3, and a light chain variable region comprising CDR1 having the amino acid sequence of SEQ ID NO: 4, CDR2 having the amino acid sequence of SEQ ID NO: 5, and CDR3 having the amino acid sequence of SEQ ID NO: 6;
(2) an antibody comprising a heavy chain variable region having the amino acid sequence of SEQ ID NO: 7 and a light chain variable region having the amino acid sequence of SEQ ID NO: 8; or
(3) an antibody comprising a heavy chain having the amino acid sequence of SEQ ID NO: 9 and a light chain having the amino acid sequence of SEQ ID NO: 10.
